# EUROPEAN PATENT APPLICATION

(11) **EP 4 113 119 A1**
(43) Date of publication of application: **04.01.2023**
(21) Application number: 21760051.9
(22) Date of filing: 09.02.2021
(51) Int. Cl.: G01N 33/574

(54) **METHOD AND REAGENT FOR DETECTING MALIGNANT OVARIAN TUMORS**

(30) Priority: 26.02.2020 JP 2020030210
(71) Applicant: Public University Corporation Yokohama City University, Yokohama-shi, Kanagawa 236-0027 (JP); Tosoh Corporation, Yamaguchi 746-8501 (JP)
(72) Inventor: MIYAGI, Etsuko, Yokohama-shi, Kanagawa 236-0004 (JP); ARAKAWA, Noriaki, Yokohama-shi, Kanagawa 236-0004 (JP); OHTAKE, Norihisa, Ayase-shi, Kanagawa 252-1123 (JP); MYOBA, Shohei, Ayase-shi, Kanagawa 252-1123 (JP)
(74) Representative: Novagraaf International SA
(86) International application number: PCT/JP2021/004736
(87) International publication number: WO 2021/172000

(57) **Abstract**

An object of the present invention is to provide a method for detecting malignant ovarian tumor as distinguished from benign ovarian tumor, and a reagent that can be used for the method. Provided are a method for detecting malignant ovarian tumor (excluding high-grade serous carcinoma) as distinguished from benign ovarian tumor, characterized by measuring the amount of TFPI2 in a sample from a patient, and a reagent for detecting malignant ovarian tumor (excluding high-grade serous carcinoma) as distinguished from benign ovarian tumor contains an antibody that specifically recognizes TFPI2 processing polypeptide and intact TFPI2.

## Description

### TECHNICAL FIELD

The present invention relates to a method and a reagent for detecting malignant ovarian tumors in which a tissue factor pathway inhibitor 2 (TFPI2) is measured.

### BACKGROUND ART

Ovarian cancer is estimated to affect about 280,000 people worldwide and about 10,000 people in Japan as new patients (GLOBOCAN 2018), and is the cancer that causes the most deaths among gynecologic malignant tumors. The current diagnosis of ovarian cancer is mainly based on diagnostic imaging and CA125, and malignancy/benignity is estimated by blood tests using a plurality of markers such as CA19-9. In the case of suspicion of malignancy, definitive diagnosis is performed by surgical ovariectomy and pathological examination of the excised lesion. However, although CA125, which is a typical ovarian cancer marker, has excellent sensitivity in detecting ovarian cancer, it may fluctuate significantly in menstruation, peritonitis, and benign tumors including endometriosis. As a result, there are problems such as delay in treatment of ovarian cancer cases, overtreatment of benign diseases due to false positive determination of the diseases, and accompanying decrease in QOL of patients. Therefore, the development of a new ovarian cancer marker that complements CA125 is underway.

Human epididymis protein 4 (HE4) is a novel ovarian cancer marker that has been covered by insurance in Japan since 2017. Although HE4 is less sensitive than CA125, it is considered to be a marker having high specificity with a low positive rate in endometriosis and other benign diseases. Furthermore, since HE4 and CA125 have a low correlation, it is said that the accuracy of distinguishing between benign and malignant ovarian tumors will be further improved by obtaining the risk of ovarian malignancy algorithm (ROMA) from the measurement values of both markers, menopausal information, and the like (Non Patent Literature 1). However, it has been pointed out that the measurement values of CA125 that has been commercialized by various in-vitro diagnostic drug companies differ from company to company. One of the reasons is the difference in the antibody used by each company for the measurement reagent and the standard used for concentration calculation, and there is an issue that it is necessary to consider the difference between the reagents of the CA125 measurement value in order to calculate the accurate ROMA. Therefore, a simple and accurate method for detecting malignant ovarian tumors by a blood test with less burden on the patient is eagerly desired.

Tissue factor pathway inhibitor 2 (TFPI2) is the same protein as placental protein 5 (PP5), and is a placentaderived serine protease inhibitor having three Kunitz-type protease inhibitor domains. It has been elucidated that TFPI2 is specifically produced from the clear cell carcinoma cell line of ovarian cancer, and that an increased gene expression of TFPI2 in an ovarian cancer patient tissue specifically occurs only in patients with clear cell carcinoma (Patent Literature 1). A method for detecting ovarian clear cell carcinoma by measuring TFPI2 in blood has been disclosed (Patent Literatures 2 and 3, Non Patent Literatures 2 and 3).

Meanwhile, another research group reported as a result of proteomics analysis of plasma of high-grade serous carcinoma (HGSC), which is one of the malignant ovarian tumors, and benign ovarian tumor that TFPI2 is one of the proteins whose median measurement value is increased in the HGSC group (Non Patent Literature 4). Nevertheless, to date, it has been unclear whether TFPI2 can be applied to distinguish malignant ovarian tumors with various histological types including borderline malignant tumor from benign ovarian tumors.

### PRIOR ART DOCUMENTS

### Patent Literatures

Patent Literature 1: Japanese Patent 5224309
Patent Literature 2: Japanese Patent 6074676
Patent Literature 3: WO2016/084912

### Non Patent Literatures

Non Patent Literature 1: Tumor Biology, 36.2 (2015), 1045-1053
Non Patent Literature 2: J. Proteome Res., 2013, 12(10), 4340-4350
Non Patent Literature 3: PloS one 11.10 (2016): e0165609.
Non Patent Literature 4: Frontiers in oncology 9(2019): 1150

### SUMMARY OF THE INVENTION

### PROBLEMS TO BE SOLVED BY THE INVENTION

An object of the present invention is to provide a method for detecting malignant ovarian tumor as distinguished from benign ovarian tumor, and a reagent that can be used for the method.

### MEANS FOR SOLVING THE PROBLEMS

The present inventors intensively studied, and found that blood TFPI2 is significantly increased in malignant ovarian tumor patients compared with benign ovarian tumor patients, and arrived at an idea that TFPI2 can detect malignant ovarian tumor with high specificity, thereby completing the present invention.

That is, the present invention includes the following embodiments.
[1] A method for detecting malignant ovarian tumor (excluding high-grade serous carcinoma) as distinguished from benign ovarian tumor, the method comprising measuring the amount of TFPI2 in a sample.
[2] The method according to [1], wherein malignant ovarian tumor (excluding high-grade serous carcinoma) is detected when the measured amount of TFPI2 exceeds a predetermined standard value.
[3] The method according to [1] or [2], wherein the amount of TFPI2 is the total of the amount of TFPI2 processing polypeptide and the amount of intact TFPI2.
[4] The method according to any one of [1] to [3], wherein the amount of TFPI2 is measured by an antigen-antibody reaction using an antibody that binds to an antigenic determinant in the region from the 23rd residue aspartic acid to the 131st residue histidine or the 130th residue cysteine of the amino acid sequence of SEQ ID No:1.
[5] The method according to [4], wherein the antibody is an antibody that recognizes Kunitz domain 1 of TFPI2.
[6] The method according to any one of [1] to [3], wherein measurement is carried out using mass spectrometry.
[7] The method according to any one of [1] to [6], which is further carried out in combination with a method for detecting an ovarian cancer marker other than TFPI2.
[8] A reagent for detecting malignant ovarian tumor (excluding high-grade serous carcinoma) as distinguished from benign ovarian tumor, comprising an antibody that binds to an antigenic determinant in the region from the 23rd residue aspartic acid to the 131st residue histidine or to the 130th residue cysteine in the amino acid sequence of SEQ ID NO:1.

### EFFECT OF THE INVENTION

The present invention provides a simple and highly accurate method for detecting malignant ovarian tumor as distinguished from benign ovarian tumor and a reagent that can be utilized for the method.

### BRIEF DESCRIPTION OF THE DRAWINGS

Fig. 1: The figure is a diagram showing TFPI2 or CA125 measurement values in a benign ovarian tumor group and a malignant ovarian tumor group in box plot. The ordinate represents the amount of TFPI2 or CA125 in blood.
Fig. 2: The figure is a diagram showing a receiver operating characteristic (ROC) curve of a benign ovarian tumor group and a malignant ovarian tumor group.
Fig. 3: The figure is a diagram showing the correlation between the TFPI2 measurement value and the CA125 measurement value in benign ovarian tumor group and a malignant ovarian tumor group. The ordinate represents the CA125 measurement value, and the abscissa represents the TFPI2 measurement value.
Fig. 4: The figure is a diagram showing TFPI2 or CA125 measurement values in a benign ovarian tumor group and a malignant ovarian tumor group in box plot. The ordinate represents the amount of TFPI2 or CA125 in blood.
Fig. 5: The figure is a diagram showing an ROC curve of a benign ovarian tumor group and a malignant ovarian tumor group.
Fig. 6: The figure is a diagram showing the correlation between the TFPI2 measurement value and the CA125 measurement value in benign ovarian tumor group and a malignant ovarian tumor group. The ordinate represents the CA125 measurement value, and the abscissa represents the TFPI2 measurement value.

### MODE FOR CARRYING OUT THE INVENTION

### <1> The method for detecting malignant ovarian tumor (excluding high-grade serous carcinoma) as distinguished from benign ovarian tumor of the present invention

A first embodiment of the present invention is a method for detecting malignant ovarian tumor (excluding high-grade serous carcinoma) as distinguished from benign ovarian tumor, which includes measuring the amount of TFPI2 in a sample. This is a method based on the fact that TFPI2 is increasingly present in a biological sample such as blood of malignant ovarian tumor as compared with benign ovarian tumor. The measurement of the amount of TFPI2 in a sample is usually performed in vitro. By this method, malignant ovarian tumor can be detected as distinguished from benign ovarian tumor with high specificity, as shown in the Examples described below.

The method of the present invention includes up to a step of detecting malignant ovarian tumor as distinguished from benign ovarian tumor, and does not include the final act of determining the diagnosis of malignant ovarian tumor. A doctor diagnoses malignant ovarian tumor or makes a treatment policy by referring to a detection result and the like by the method of the present invention.

Malignant ovarian tumors detected in the present invention refer to malignant tumors and borderline malignant tumors. Various tumors included therein are targeted and are not particularly limited except for high-grade serous carcinoma.

Examples of malignant tumors include: epithelial tumors such as non-invasive low-grade serous carcinoma, low-grade serous carcinoma, mucinous carcinoma, endometrioid carcinoma, clear cell carcinoma, malignant Brenner tumor, seromucinous carcinoma, undifferentiated carcinoma (low-grade endometrioid stromal sarcoma, high-grade endometrioid stromal sarcoma) and mixed epithelial and mesenchymal tumors (adenosarcoma, carcinosarcoma); sex cord-stromal tumors such as pure stromal tumors (cellular fibroma, fibrosarcoma, malignant steroid cell tumor) and pure sex cord tumors (adult granulosa cell tumor, juvenile granulosa cell tumor, Sertoli cell tumor, sex cord tumor with annular tubules); mixed sex cord-stromal tumors such as Sertoli-Leydig cell tumor and other sex cord-stromal tumors; germ cell tumors such as undifferentiated germ cell tumor/dysgerminoma, yolk sac tumor, embryonal carcinoma (embryonic carcinoma), polyembryoma, immature teratoma (G3), mature teratoma with malignant transformation, fibrosarcoma, choriocarcinoma, and mixed germ cell tumor; and carcinoma and sarcoma; malignant lymphoma; and secondary (metastatic) tumor. Of these, clear cell carcinoma, low-grade serous carcinoma, endometrioid carcinoma, and mucinous carcinoma can be mentioned as preferable examples. Further, clear cell carcinoma, endometrioid carcinoma, and mucinous carcinoma can be mentioned as more preferable examples.

Examples of borderline malignant tumors include: epithelial tumors such as serous borderline tumor, serous borderline tumor with micropapillary pattern, mucinous borderline tumor, endometrioid borderline tumor, clear cell borderline tumor, adenofibroma, surface papilloma, borderline Brenner tumor, and seromucinous borderline tumor; sex cord-stromal tumors such as granulosa cell tumor, Sertoli-stromal cell tumor (moderately differentiated), steroid cell tumor (unclassified), and gynandroblastoma; and germ cell tumors such as immature teratoma (G1/G2), carcinoid, and strumal carcinoid; and gonadoblastoma (pure).

TFPI2 measured in the present invention is not particularly limited, and may be, for example, intact TFPI2 (hereinafter, also referred to as "I-TFPI2") and/or TFPI2 processing polypeptide (hereinafter, also referred to as "NT-TFPI2") .

SEQ ID NO:1 shows an amino acid sequence based on the human TFPI2 cDNA. In SEQ ID NO:1, a signal peptide extends from the initiation methionine to the 22nd residue glycine.

"Intact TFPI2" means the peptide represented by the 23rd to 235th residues in the amino acid sequence of SEQ ID NO:1.

"NT-TFPI2" means a peptide fragment containing a Kunitz domain 1 located on the N-terminal side of intact TFPI2, as described in Patent Document 3. More specifically, NT-TFPI2 is a peptide containing at least a sequence from the 23rd residue aspartic acid to the 131st residue histidine or the 130th residue cysteine of the amino acid sequence of SEQ ID NO:1 or a peptide containing an amino acid sequence having 80% or more identity with the above-described sequence. The identity is preferably not less than 90%, more preferably not less than 95%. The polypeptide of the present invention may be a polypeptide having an amino acid sequence which is the same as the above sequence except that one or several amino acids are deleted, substituted, inserted, and/or added. The term "several" means preferably 2 to 20, more preferably 2 to 10, and still more preferably 2 to 5. Although NT-TFPI2 may also have other peptide fragments on both sides of the above sequence, it preferably does not have an antigenic determinant (epitope) for an antibody that recognizes Kunitz domain 3 of TFPI2.

Examples of the patient-derived sample (test sample) in the present invention include blood components such as whole blood, blood cells, serum, and plasma, cell or tissue extract, urine, cerebrospinal fluid, abdominal cavity lavage fluid, and ascites. An ovarian tissue biopsy sample may be used as a test target, and in that case, an extract of the biopsy sample or a culture supernatant is measured. It is preferable to use a body fluid such as a blood component or urine as a sample because a test can be performed easily and non-invasively, and considering the ease of collecting a sample and the versatility of other test items, it is particularly preferable to use a blood component as a sample. The sample dilution ratio may be appropriately selected from undiluted to 100-fold diluted according to the type and state of the sample to be used.

In the method for detecting malignant ovarian tumor (excluding high-grade serous carcinoma) as distinguished from benign ovarian tumor of the present invention, A method for detecting TFPI2 and a method for detecting another tumor marker of ovarian cancer (an ovarian cancer marker other than TFPI2, also referred to as "another ovarian cancer marker") may be used in combination. The combination is not particularly limited. Examples of the combination of a method for detecting TFPI2 and a method for detecting another ovarian cancer marker in the method for detecting malignant ovarian tumor (excluding high-grade serous carcinoma) as distinguished from benign ovarian tumor of the present invention include:
(A) a method for detecting malignant ovarian tumor (excluding high-grade serous carcinoma) as distinguished from benign ovarian tumor by performing a method for detecting TFPI2 and a method for detecting another ovarian cancer marker simultaneously or separately for a sample to be measured;
(B) a method for detecting malignant ovarian tumor (excluding high-grade serous carcinoma) as distinguished from benign ovarian tumor by a method in which a method for detecting TFPI2 is first applied to a sample to be measured, and then another ovarian cancer marker is detected in a sample that has been determined to be negative as a result of the detection; and
(C) a method for detecting malignant ovarian tumor (excluding high-grade serous carcinoma) as distinguished from benign ovarian tumor by a method in which a method for detecting another ovarian cancer marker is first applied to a sample to be measured, and then TFPI2 is detected in a sample that has been determined to be negative as a result of the detection. The method (B) or (C) is preferable in that the reagent used for detection is not wasted.

In the method for detecting malignant ovarian tumor (excluding high-grade serous carcinoma) as distinguished from benign ovarian tumor of the present invention, another ovarian cancer marker to be detected may be selected as appropriate from conventionally known markers. Examples thereof include Cancer Antigen 125 (CA125), Cancer Antigen 546 (CA546), Cancer Antigen 72-4 (CA72-4), Cancer Antigen 130 (CA130), Cancer Antigen 602 (CA602), Sialyl Tn antigen (SLN), galactosyltransferase associated with tumor (GAT), lysophosphatidic acid (LPA), and human epididymis protein 4 (HE4). Of these, CA125, which is the most widely used ovarian cancer marker, is preferable as another ovarian cancer marker that is used in the method for detecting malignant ovarian tumor (excluding high-grade serous carcinoma) as distinguished from benign ovarian tumor of the present invention from the viewpoint that clinical usefulness of CA125 has been established. Another ovarian cancer marker detected by the method for detecting malignant ovarian tumor (excluding high-grade serous carcinoma) as distinguished from benign ovarian tumor of the present invention may be only one kind or two or more kinds thereof.

The sampling time of the sample in the present invention is not particularly limited. For example, a sample can be collected at any time from a preoperative period when malignant ovarian tumor is suspected due to pelvic mass confirmed by image diagnosis or the like and detailed examination is performed to a follow-up period after definitive diagnosis of malignant ovarian tumor by postoperative pathological examination, and any sample collected at any stage such as before and after definitive diagnosis and before and after the start of treatment can be used in the method of the present invention.

In the detection method of the present invention, it is preferable to determine that malignant ovarian tumor (excluding high-grade serous carcinoma) is detected as distinguished from benign ovarian tumor when the amount of TFPI2 obtained by the measurement exceeds a preset standard value (Cutoff value). Here, the TFPI2 amount may be either an intact TFPI2 amount, an NT-TFPI2 amount, or a total of an intact TFPI2 amount and an NT-TFPI2 amount, and the total of the intact TFPI2 amount and the NT-TFPI2 amount is more preferable from the viewpoint of achieving both easy measurement and sufficient sensitivity and specificity.

The standard value used for the determination may be either the measurement value or the converted concentration value. The converted concentration value means a value converted from the measurement value based on a calibration curve prepared using TFPI2 as a standard sample.

The standard value (Cutoff value) for determining malignant ovarian tumor (excluding high-grade serous carcinoma) as distinguished from benign ovarian tumor can be appropriately set to a measurement value showing optimum sensitivity and specificity by measuring benign ovarian tumor and malignant ovarian tumor (excluding high-grade serous carcinoma) and analyzing receiver operating characteristic (ROC) curve.

Hereinafter, a method for measuring TFPI2 will be described.

In the present invention, the amount of NT-TFPI2 or the amount of intact TFPI2 in a sample may be individually measured, or the values may be summed to obtain the total amount. The total amount of NT-TFPI2 and intact TFPI2 in a sample may be measured with a measurement system capable of measuring at one time. Alternatively, as described later, the amount of NT-TFPI2 may be indirectly measured from the total amount of both measurements and the measured amount of intact TFPI2 alone.

In the method of the present invention, the method for measuring the amount of NT-TFPI2 and/or the amount of intact TFPI2 is not limited. Examples of the method include methods utilizing antigen-antibody reaction in which an antibody that recognizes NT-TFPI2 and/or intact TFPI2 is used, and methods utilizing mass spectrometry.

(a) A competition method using a labeled measuring object and an antibody that recognizes the measuring object, which method utilizes competitive binding of the labeled measuring object and the measuring object contained in the sample to the antibody.
(b) A method using surface plasmon resonance, wherein the sample is brought into contact with a chip on which an antibody that recognizes the measuring object is immobilized, and a signal dependent on binding of the antibody to the measuring object is detected.
(c) A fluorescence polarization immunoassay using a fluorescently labeled antibody that recognizes a measuring object, which immunoassay utilizes the phenomenon that binding of the antibody to the measuring object causes an increase in the degree of fluorescence polarization.
(d) A sandwich method using two kinds of antibodies (one of which is a labeled antibody) that recognize the measuring object at different epitopes, wherein formation of a complex of the three molecules, that is, the two antibodies and the measuring object, is allowed to occur.
(e) A method in which pretreatment is carried out by concentrating the measuring object in the sample using an antibody that recognizes the measuring object, and then the measuring object is separated from the antibody and detected using a mass spectrometer or the like.

Although the methods (d) and (e) are simple and versatile, the method (d) is preferable for processing of a large number of samples since the technologies related to the reagents and the devices for this method have been sufficiently established.

Specific examples of the methods for measuring the amount of NT-TFPI2 and/or the amount of intact TFPI2 utilizing antigen-antibody reaction include the following.
(A) A method using an antibody that recognizes both NT-TFPI2 and intact TFPI2, wherein the total amount of NT-TFPI2 and intact TFPI2 is measured (NT+I-TFPI2 assay system). The antibody that recognizes both NT-TFPI2 and intact TFPI2 is preferably an antibody that binds to an antigenic determinant in the region from the 23rd residue aspartic acid to the 131st residue histidine or to the 130th residue cysteine in the TFPI2 amino acid sequence represented by SEQ ID NO:1. The antibody is more preferably an antibody that recognizes Kunitz domain 1 of TFPI2 as an antigenic determinant. In cases where the above-mentioned sandwich method is used in this method, two kinds of antibodies for different epitopes are used as the antibody.
(B) A method using an antibody that does not recognize NT-TFPI2 but recognizes intact TFPI2, wherein the amount of intact TFPI2 alone is measured (I-TFPI2 assay system). The antibody that does not recognize NT-TFPI2 but recognizes intact TFPI2 is preferably an antibody that recognizes Kunitz domain 3 of TFPI2 as an antigenic determinant. In cases where the above-mentioned sandwich method is used in this method, two kinds of antibodies for different epitopes are used as the antibody. At least one of these is an antibody that does not recognize NT-TFPI2 but recognizes intact TFPI2, and the other may be either an antibody that does not recognize NT-TFPI2 but recognizes intact TFPI2, or an antibody that recognizes both NT-TFPI2 and intact TFPI2.
(C) A method in which the amount of intact TFPI2 alone measured in the I-TFPI2 assay system of (B) is subtracted from the total amount of NT-TFPI2 and intact TFPI2 measured in the NT+I-TFPI2 assay system of (A), to calculate the amount of NT-TFPI2 alone.
(D) A method using an antibody that does not recognize intact TFPI2 but recognizes NT-TFPI2, wherein the amount of NT-TFPI2 alone is measured. Examples of the antibody that does not recognize intact TFPI2 but recognizes NT-TFPI2 include antibodies that specifically recognize a peptide sequence in the C-terminal portion of NT-TFPI2. For example, in cases where the above-mentioned sandwich method is used, such an antibody is used as the solidphase antibody, and an antibody that recognizes Kunitz domain 1 as an antigenic determinant is used as the detection antibody.

In the method for detecting malignant ovarian tumor (excluding high-grade serous carcinoma) as distinguished from benign ovarian tumor of the present invention, the amount of NT-TFPI2 alone measured by the method of (C) or (D) may be used as a criterion. However, sufficient sensitivity and specificity can be obtained also by using the total amount of NT-TFPI2 and intact TFPI2 measured by the method of (A) as a criterion. The latter method is preferable from the viewpoint of the fact that the antibody can be easily obtained, and that the measurement can be simply carried out by a single step.

An antibody that recognizes NT-TFPI2 and/or intact TFPI2 can be obtained by immunizing an animal using as an immunogen, for example, an NT-TFPI2 polypeptide or protein, an oligopeptide composed of a partial region of the intact TFPI2 polypeptide or the TFPI2 protein, or a polynucleotide encoding the intact or a partial region of the NT-TFPI2 polypeptide or of the TFPI2 protein. The protein or the oligopeptide or polypeptide may not reflect the three-dimensional structure of TFPI2 in a living body, or the structure thereof may change during a preparation process. Therefore, an obtained antibody may not have high specificity or binding capacity to TFPI2 in a desired living body, and even when a measurement system is constructed using the present antibody, the TFPI2 concentration contained in a sample as a result may not be accurately quantified.

On the other hand, when an expression vector containing a polynucleotide encoding an intact or partial region of a TFPI2 polypeptide or an intact TFPI2 protein is used as an immunogen, the intact or partial region of the TFPI2 polypeptide or intact TFPI2 protein is expressed in an immunized animal, an immune response is elicited, and an antibody having high specificity and binding capacity (that is, high affinity) to the TFPI2 in a sample is obtained, which is preferable.

The animal to be used for the immunization is not limited as long as the animal has ability to produce antibodies. The animal may be a mammal normally used for immunization, such as a mouse, a rat, or a rabbit, or may be a bird such as a chicken.

In blood, there is also TFPI1, known as a homologue of TFPI2. Therefore, it is desirable to use an antibody that does not cross with TFPI1 but specifically recognizes only TFPI2.

The antibody that recognizes TFPI2 may be either a monoclonal antibody or a polyclonal antibody. The antibody is preferably a monoclonal antibody.

The method of establishment of a hybridoma cell that produces an antibody that recognizes TFPI2 may be appropriately selected from methods whose techniques have been established. For example, a hybridoma cell that produces a monoclonal antibody that recognizes TFPI2 can be established by collecting B cells from an animal immunized by the above method, fusing the B cells with myeloma cells electrically or in the presence of polyethylene glycol, selecting a hybridoma cell that produces a desired antibody using HAT medium, and preparing the selected hybridoma cell into a monoclone by the limiting dilution method.

The selection of the monoclonal antibody that recognizes TFPI2 used in the present invention may be carried out based on affinity to GPI (glycosylphosphatidylinositol)-anchor type TFPI2 or secretory TFPI2 derived from a host expression system.

The host is not limited, and may be appropriately selected from microorganisms such as E. coli or yeast, insect cells, and animal cells that are usually used for protein expression by those skilled in the art. The host is preferably a mammalian cell since it enables the expression of a protein having a structure similar to that of natural TFPI2 by post-translational modification such as disulfide bonding or glycosylation. Examples of the mammalian cell include the human embryonic kidney (HEK)-derived 293T cell line, monkey kidney COS7 cell line, Chinese hamster ovary (CHO) cells, and cancer cells isolated from humans.

The method of purification of the antibody to be used in the present invention may be appropriately selected from methods whose techniques have been established. For example, after culturing hybridoma cells which are established by the above method and which produce an antibody, the culture supernatant may be collected, and the antibody may be concentrated, if necessary, by ammonium sulfate precipitation. Thereafter, by affinity chromatography using a carrier to which Protein A, Protein G, Protein L, or the like is immobilized, and/or by ionexchange chromatography, purification of the antibody is possible.

The labeled antibody used for the antigen-antibody reaction in the sandwich method described above may be prepared by labeling an antibody purified by the above method with an enzyme such as peroxidase or alkaline phosphatase. The labeling may also be carried out using a method whose technique has been sufficiently established.

The method for measuring the amount of TFPI2 utilizing mass spectrometry in the method of the present invention is described below concretely.

In cases of a blood sample, a pretreatment step is preferably carried out by removing major proteins contained in large amounts in blood such as albumin, immunoglobulin, and transferrin using Agilent Human 14 or the like, and performing further fractionation by ion exchange, gel filtration, reverse-phase HPLC, and/or the like. Alternatively, only TFPI2 can be specifically recovered by an immunological method using an anti-TFPI2 antibody.

The measurement can be carried out by tandem mass spectrometry (MS/MS), liquid chromatography-tandem mass spectrometry (LC/MS/MS), matrix assisted laser desorption ionization time-of-flight mass spectrometry (MALDI-TOF/MS), surface enhanced laser desorption ionization mass spectrometry (SELDI-MS), or the like.

The method for detecting malignant ovarian tumor (excluding high-grade serous carcinoma) as distinguished from benign ovarian tumor of the present invention can be applied to a method for treating malignant ovarian tumor. In other words, the present invention provides a method for treating malignant ovarian tumor in a patient, the method comprising:
(i) a step in which a patient is identified as one in which a measurement value of the TFPI2 amount exceeds a preset standard value; and
(ii) a step in which a treatment is provided to the patient identified as above.

In the identification of the step (i), the measurement of the amount of TFPI2 may be performed by using an antibody that specifically recognizes NT-TFPI2 and/or intact TFPI2 or by mass spectrometry.

Examples of the treatment of the step (ii) include surgical treatment, drug therapy, and radiation therapy, but are not particularly limited.

### <2> The reagent for detecting malignant ovarian tumor (excluding high-grade serous carcinoma) as distinguished from benign ovarian tumor of the present invention

A second embodiment of the present invention is a reagent for detecting malignant ovarian tumor (excluding high-grade serous carcinoma) as distinguished from benign ovarian tumor, which contains an antibody that recognizes NT-TFPI2 and/or intact TFPI2. Such an antibody is more preferably an antibody that recognizes NT-TFPI2 and intact TFPI2. More specifically, an antibody that binds to an antigenic determinant in the region from the 23rd residue aspartic acid to the 131st residue histidine or to the 130th residue cysteine in the amino acid sequence of SEQ ID NO:1 is still more preferable.

In cases where the reagent of the present invention is used in the sandwich method described above, the reagent preferably contains, as the antibody, two kinds of antibodies for different epitopes.

The antibody contained in the reagent of the present invention may be an antibody itself, a labeled antibody, or an antibody immobilized on a solid phase.

The reagent of the present invention is described below concretely for cases where it is used for a two-step sandwich method, which is one embodiment of the sandwich method. However, the present invention is not limited thereto.

The reagent of the present invention can be prepared by the method described in the following (I) to (III).
(I) First, Antibody 1, one of the two kinds of antibodies for different epitopes that recognize TFPI2 (hereinafter referred to as "Antibody 1" and "Antibody 2"), is bound to a carrier capable of B/F (Bound/Free) separation such as an immunoplate or magnetic particles. The binding method may be either physical binding utilizing hydrophobic bonding, or chemical bonding using a linker reagent capable of cross-linking two substances to each other.
(II) After the binding of the Antibody 1 to the carrier, the carrier surface is subjected to blocking treatment using bovine serum albumin, skim milk, a commercially available immunoassay blocking agent, chemically synthesized polymer for suppressing protein adsorption, or the like for preventing non-specific binding, to provide a primary reagent.
(III) After labeling the other antibody, Antibody 2, a solution containing the obtained labeled antibody is provided as a secondary reagent. Preferred examples of the substance with which Antibody 2 is labeled include enzymes such as peroxidase and alkaline phosphatase; substances detectable with detection devices, such as fluorescent substances, chemiluminescent substances, and radioisotopes; and substances to which another molecule specifically binds, such as biotin, to which avidin specifically binds. Preferred examples of the solution for the secondary reagent include buffers with which antigen-antibody reaction can be favorably carried out, such as phosphate buffer and Tris-HCl buffer. The thus prepared reagent of the present invention may be freezedried, if necessary.

In cases of a one-step sandwich method, binding of Antibody 1 to the carrier and subsequent blocking treatment may be carried out in the same manner as in (I) and (II) to prepare an antibody-immobilized carrier, and a buffer containing a labeled Antibody 2 may be further added to the antibody-immobilized carrier, to provide a reagent.

For measurement of TFPI2 by a two-step sandwich method using reagents obtained by the method described above, the method described in the following (IV) to (VI) may be carried out.

(IV) The primary reagent prepared in (II) is brought into contact with a sample for a predetermined period of time at a constant temperature. In terms of the reaction conditions, the reaction may be carried out at a temperature within the range of 4°C to 40°C for 5 minutes to 180 minutes.

(V) Unreacted substances are removed by B/F separation, and then the secondary reagent prepared in (III) is brought into contact with the resulting reaction product for a predetermined period of time at a constant temperature to allow the formation of a sandwich complex. In terms of the reaction conditions, the reaction may be carried out at a temperature within the range of 4°C to 40°C for 5 minutes to 180 minutes.

(VI) Unreacted substances are removed by B/F separation, and the labeling substance of the labeled antibody is quantified. Based on a calibration curve prepared using a TFPI2 solution having a known concentration as a standard sample, the human TFPI2 in the sample is quantified.

The amount of each reagent component such as the antibody contained in the reagent may be appropriately set depending on conditions such as the amount of the sample, the type of the sample, the type of the reagent, and the measurement method. More specifically, for example, in cases where the amount of TFPI2 is measured as described below by a sandwich method using 20 µL of serum or plasma as a sample, the amount of the antibody to be bound to the carrier may be 100 ng to 1000 µg, and the amount of the labeled antibody may be 2 ng to 20 µg per the reaction system in which 20 µL of the sample is reacted with the antibodies.

The reagent of the present invention is applicable to either manual measurement or measurement using an automatic immunodiagnostic device. Measurement using an automatic immunodiagnostic device is especially preferable since it enables the measurement without being influenced by endogenous measurement-inhibiting factors and competing enzymes contained in the sample, and also enables rapid quantification of the concentration of TFPI2.

Another aspect of the present invention is the use of NT-TFPI2 and/or intact TFPI2 in producing a reagent for detecting malignant ovarian tumor (excluding high-grade serous carcinoma) as distinguished from benign ovarian tumor.

Another aspect of the present invention is the use of NT-TFPI2 and/or intact TFPI2 in detecting malignant ovarian tumor (excluding high-grade serous carcinoma) as distinguished from benign ovarian tumor.

Another aspect of the present invention is NT-TFPI2 and/or intact TFPI2 used for detecting malignant ovarian tumor (excluding high-grade serous carcinoma) as distinguished from benign ovarian tumor.

Another aspect of the present invention is the use of an antibody that recognizes NT-TFPI2 and/or intact TFPI2 in producing a reagent for detecting malignant ovarian tumor (excluding high-grade serous carcinoma) as distinguished from benign ovarian tumor.

Another aspect of the present invention is the use of an antibody that recognizes NT-TFPI2 and/or intact TFPI2 in detecting malignant ovarian tumor (excluding high-grade serous carcinoma) as distinguished from benign ovarian tumor.

Another aspect of the present invention is an antibody that recognizes NT-TFPI2 and/or intact TFPI2 used for detecting malignant ovarian tumor (excluding high-grade serous carcinoma) as distinguished from benign ovarian tumor.

As the antibody in these aspects, an antibody that binds to an antigenic determinant in the region from the 23rd residue aspartic acid to the 131st residue histidine or to the 130th residue cysteine in the amino acid sequence of SEQ ID NO:1 is preferable.

### EXAMPLES

Examples are shown below for concrete description of the present invention. However, these Examples merely show examples of the present invention, and the present invention is not limited by Examples.

### <Example 1> Preparation of Reagent for TFPI2 Measurement

According to the method of Patent Document 3, a reagent for TFPI2 measurement was prepared using a TFPI2 antibody obtained by the DNA immunization method as follows.
(1) Physical adsorption of an anti-TFPI2 monoclonal antibody (TS-TF04) to water-insoluble ferrite-containing carriers was allowed at room temperature for one day and night such that the adsorption occurred at 100 ng/carrier, and blocking was then carried out with 100 mM Tris buffer (pH 8.0) supplemented with 1% BSA at 53°C for 4 hours, to prepare anti-TFPI2 antibody-immobilized carriers.
(2) An alkaline phosphatase labeled anti-TFPI2 antibody was prepared with anti-TFPI2 monoclonal antibodies (TS-TF01) using an alkaline phosphatase labeling kit (manufactured by Dojindo Laboratories).
(3) In each of magnetic force-permeable containers (volume, 1.2 mL), 12 antibody-immobilized carriers prepared in (1) were placed, and 100 µL of a buffer (Tris buffer supplemented with 3% BSA, pH 8.0) supplemented with 1 µg/mL of an alkaline phosphatase labeled antibody prepared in (2) was added thereto, followed by carrying out freeze-drying, to prepare a TFPI2 assay reagent. The TFPI2 assay reagents prepared were tightly closed and sealed under nitrogen gas, and stored at 4°C until the assay.

### <Example 2> Evaluation of Clinical Samples

Table 1 shows the breakdown list for the clinical samples used in this example. 33 cases of benign ovarian tumor serum and 38 cases of malignant ovarian tumor serum (excluding high-grade serous carcinoma) were samples collected by the same protocol at Yokohama City University Obstetrics & Gynecology Department, and were provided with informed consent and the approval of the Yokohama City University Ethics Committee.

**[Table 1]**

| | | Number of cases | |
|---|---|---|---|
| Benign ovarian tumor | Endometriosis | 14 | 33 |
| | Non-endometriosis | 19 | |
| Malignant ovarian tumor | Borderline malignant tumor | 12 | 38 |
| | Clear cell carcinoma | 13 | |
| | Endometrioid carcinoma | 4 | |
| | Mucinous carcinoma | 4 | |
| | Others | 5 | |

As an evaluation device, a fully automatic enzyme immunoassay device AIA-2000 (manufactured by Tosoh Corporation; manufacturing/marketing notification number, 13B3X90002000009) was used. The measurement of TFPI2 by the fully automatic enzyme immunoassay device AIA-2000 was carried out by the following procedure:
(1) by automatically dispensing 20 µL of a sample and 80 µL of a diluent containing a surfactant to a container storing a TFPI2 assay reagent prepared in Example 1;
(2) carrying out antigen-antibody reaction at a constant temperature of 37°C for 10 minutes;
(3) carrying out eight times of washing using a buffer containing a surfactant after B/F separation; and
(4) adding 4-methylumbelliferyl phosphate to the container, the concentration of 4-methylumbelliferone produced by alkaline phosphatase per unit time was provided as the measurement value (TFPI2 intensity, nmol/(L·s)).

A calibration curve was prepared using a commercially available TFPI2 recombinant protein (R&D Systems, Inc.) as a standard, and the TFPI2 concentration in the sample was calculated. The measurement of CA125 was carried out using the E-test TOSOH II CA125 assay reagent (manufactured by Tosoh Corporation; approval number: 20700AMZ00504000).

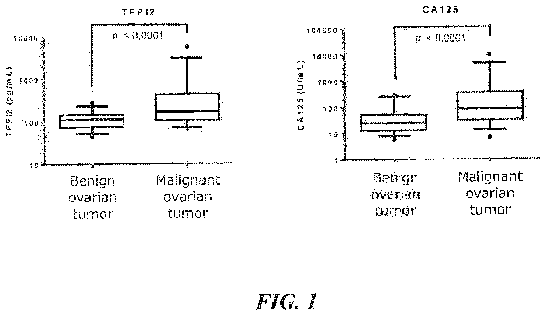

BoxPlot of blood TFPI2 values and blood CA125 measurement values is shown in Fig. 1. It was revealed that both TFPI2 and CA125 showed a high value with a statistically significant difference in malignant ovarian tumor (excluding high-grade serous carcinoma) compared with benign ovarian tumor (Mann-Whitney U test, p < 0.0001).

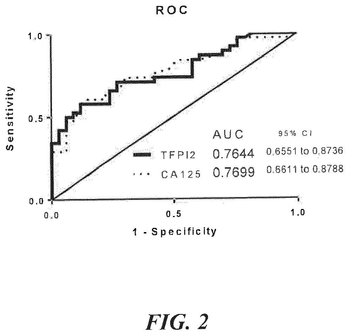

The ROC analysis result is shown in Fig. 2. The area under the curve (AUC) of TFPI2 was 0.7644 and AUC of CA125 was 0.7699, indicating that TFPI2 had favorable malignant ovarian tumor (excluding high-grade serous carcinoma) detection performance (distinguishing performance between benign ovarian tumor and malignant ovarian tumor (excluding high-grade serous carcinoma)) comparable to CA125.

### <Example 3> Comparison of Benignity vs. Malignancy Discrimination Performance between TFPI2 and CA125

The cutoff value (191 pg/mL) of TFPI2 was set to the concentration at which the Youden index (specificity + sensitivity - 1) became the maximum value from the ROC analysis results of Example 2. For CA125, the cutoff value (35 U/mL) used in clinical practice was used. Table 2 shows the distinguishing performance (sensitivity, specificity, positive predictive value, negative predictive value, positive likelihood ratio, negative likelihood ratio) of each marker calculated using the 2 × 2 contingency table. TFPI2 was shown to be superior to CA125 in specificity, positive predictive value, and positive likelihood ratio while CA125 was shown to be superior to TFPI2 in sensitivity.

**[Table 2]**

| | Value (95% confidence interval) | |
|---|---|---|
| | TFPI2 | CA125 |
| Sensitivity | 0.47 (0.33 - 0.63) | 0.74 (0.58 - 0.85) |
| Specificity | 0.94 (0.80 - 0.98) | 0.61 (0.44 - 0.75) |
| Positive predictive value | 0.90 (0.70 - 0.97) | 0.68 (0.53 - 0.80) |
| Negative predictive value | 0.61 (0.47 - 0.73) | 0.67 (0.49 - 0.81) |
| Positive likelihood ratio | 7.77 (1.96 - 31.20) | 1.87 (1.18 - 2.97) |
| Negative likelihood ratio | 0.56 (0.41 - 0.77) | 0.43 (0.24 - 0.79) |

### <Example 4> Correlation Analysis of TFPI2 and CA125

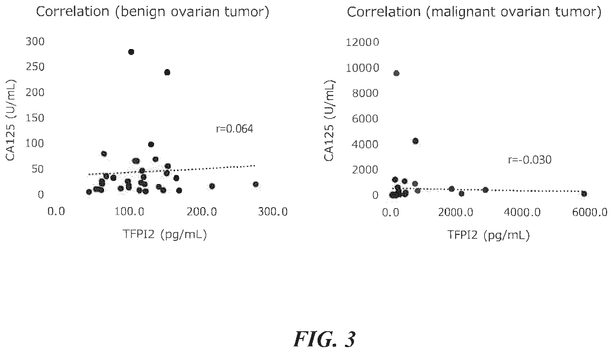

Fig. 3 shows the results of analyzing the correlation between TFPI2 and CA125 in benign ovarian tumor or malignant ovarian tumor (excluding high-grade serous carcinoma) based on the results of Example 2. No significant correlation was found between TFPI2 and CA125 in either benign ovarian tumor or malignant ovarian tumor (excluding high-grade serous carcinoma), suggesting that they are independent indicators.

### <Example 5> Evaluation of Additional Clinical Samples

Table 3 shows the breakdown list for the clinical samples used in this example. 77 cases of benign ovarian tumor serum and 274 cases of malignant ovarian tumor serum (excluding high-grade serous carcinoma) were samples collected by the same protocol at Yokohama City University Obstetrics & Gynecology Department, and were provided with informed consent and the approval of the Yokohama City University Ethics Committee.

**[Table 3]**

| Breakdown list of samples | | Number of cases | |
|---|---|---|---|
| Benign ovarian tumor | Endometriosis | 21 | 77 |
| | Non-endometriosis | 56 | |
| Malignant ovarian tumor | Borderline malignant tumor | 65 | 274 |
| | Clear cell carcinoma | 69 | |
| | Low-grade serous carcinoma | 67 | |
| | Endometrioid carcinoma | 31 | |
| | Mucinous carcinoma | 24 | |
| | Others | 18 | |

As an evaluation device, a fully automatic enzyme immunoassay device AIA-900 (manufactured by Tosoh Corporation; manufacturing/marketing notification number: 13B3X90002000012) was used. The measurement of TFPI2 by the fully automatic enzyme immunoassay device AIA-900 was carried out by the following procedure:
(1) by automatically dispensing 20 µL of a sample and 80 µL of a diluent containing a surfactant to a container storing a TFPI2 assay reagent prepared in Example 1;
(2) carrying out antigen-antibody reaction at a constant temperature of 37°C for 10 minutes;
(3) carrying out eight times of washing using a buffer containing a surfactant after B/F separation; and
(4) adding 4-methylumbelliferyl phosphate to the container, the concentration of 4-methylumbelliferone produced by alkaline phosphatase per unit time was provided as the measurement value (TFPI2 intensity, nmol/(L·s)).

A calibration curve was prepared using a commercially available TFPI2 recombinant protein (R&D Systems, Inc.) as a standard, and the TFPI2 concentration in the sample was calculated. The measurement of CA125 was carried out using the E-test "TOSOH" II CA125 assay reagent (manufactured by Tosoh Corporation; approval number: 20700AMZ00504000).

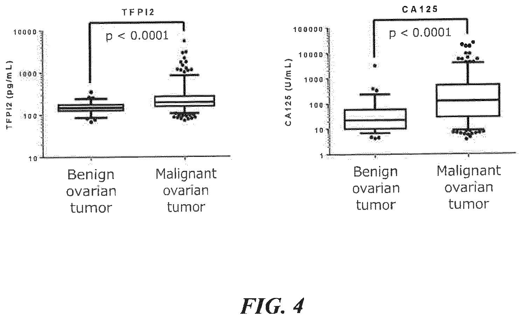

BoxPlot of blood TFPI2 values and blood CA125 measurement values is shown in Fig. 4. It was revealed that both TFPI2 and CA125 showed a high value with a statistically significant difference in malignant ovarian tumor (excluding high-grade serous carcinoma) compared with benign ovarian tumor (Mann-Whitney U test, p < 0.0001).

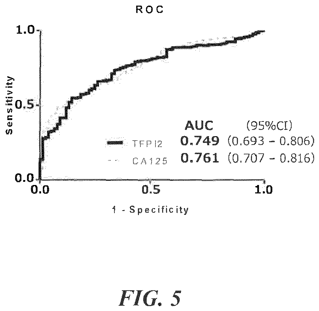

The ROC analysis result is shown in Fig. 5. The area under the curve (AUC) of TFPI2 was 0.749 and AUC of CA125 was 0.761, indicating that TFPI2 had favorable malignant ovarian tumor (excluding high-grade serous carcinoma) detection performance (distinguishing performance between benign ovarian tumor and malignant ovarian tumor (excluding high-grade serous carcinoma)) comparable to CA125.

### <Example 6> Comparison of Benignity vs. Malignancy Discrimination Performance between TFPI2 and CA125 in Additional Samples

The cutoff values for TFPI2 and CA125 were the same as in Example 3 (TFPI2: 191 pg/mL; CA125: 35U/mL). Table 4 shows the distinguishing performance (sensitivity, specificity, positive predictive value, negative predictive value, positive likelihood ratio, negative likelihood ratio) of each marker calculated using the 2 × 2 contingency table. As in Example 3, TFPI2 was shown to be superior to CA125 in specificity, positive predictive value, and positive likelihood ratio while CA125 was shown to be superior to TFPI2 in sensitivity.

**[Table 4]**

| Evaluation item | Value (95% confidence interval) | |
|---|---|---|
| | TFPI2 | CA125 |
| Sensitivity | 0.54 (0.48 - 0.60) | 0.72 (0.66 - 0.77) |
| Specificity | 0.86 (0.76 - 0.93) | 0.61 (0.49 - 0.72) |
| Positive predictive value | 0.93 (0.89 - 0.96) | 0.87 (0.84 - 0.90) |
| Negative predictive value | 0.35 (0.31 - 0.37) | 0.38 (0.32 - 0.44) |
| Positive likelihood ratio | 3.81 (2.27 - 6.71) | 1.86 (1.43 - 2.49) |
| Negative likelihood ratio | 0.53 (0.48 - 0.62) | 0.45 (0.36 - 0.59) |

### <Example 7> Correlation Analysis of TFPI2 and CA125 in Additional Samples

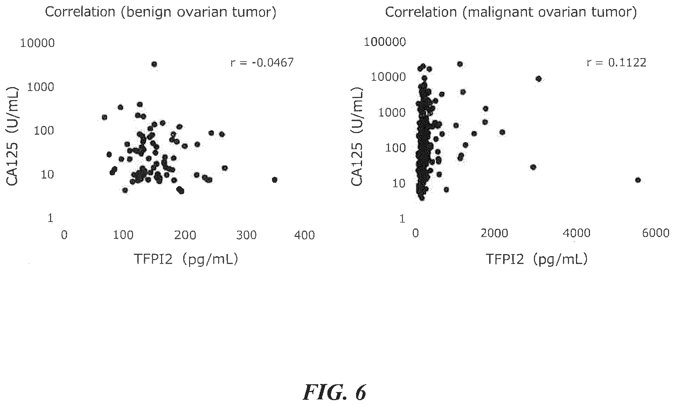

Fig. 6 shows the results of analyzing the correlation between TFPI2 and CA125 in benign ovarian tumor or malignant ovarian tumor (excluding high-grade serous carcinoma) based on the results of Example 5. No significant correlation was found between TFPI2 and CA125 in either benign ovarian tumor or malignant ovarian tumor (excluding high-grade serous carcinoma), suggesting that they are independent indicators.

### INDUSTRIAL APPLICABILITY

The present invention provides a method for detecting malignant ovarian tumor (excluding high-grade serous carcinoma) as distinguished from benign ovarian tumor by a blood test that is simple and has a relatively low burden on the patient. This is expected to contribute to the medical treatment of malignant ovarian tumor, which does not have an effective tumor marker and depends on image diagnosis, and is industrially considerably useful.

## Claims

1. A method for detecting malignant ovarian tumor (excluding high-grade serous carcinoma) as distinguished from benign ovarian tumor, said method comprising measuring the amount of TFPI2 in a sample.

2. The method according to claim 1, wherein malignant ovarian tumor (excluding high-grade serous carcinoma) is detected when said measured amount of TFPI2 exceeds a predetermined standard value.

3. The method according to claim 1 or 2, wherein said amount of TFPI2 is the total of the amount of TFPI2 processing polypeptide and the amount of intact TFPI2.

4. The method according to any one of claims 1 to 3, wherein said amount of TFPI2 is measured by an antigen-antibody reaction using an antibody that binds to an antigenic determinant in the region from the 23rd residue aspartic acid to the 131st residue histidine or the 130th residue cysteine of the amino acid sequence of SEQ ID No:1.

5. The method according to claim 4, wherein said antibody is an antibody that recognizes Kunitz domain 1 of TFPI2.

6. The method according to any one of claims 1 to 3, wherein measurement is carried out using mass spectrometry.

7. The method according to any one of claims 1 to 6, which is further carried out in combination with a method for detecting an ovarian cancer marker other than TFPI2.

8. A reagent for detecting malignant ovarian tumor (excluding high-grade serous carcinoma) as distinguished from benign ovarian tumor, comprising an antibody that binds to an antigenic determinant in the region from the 23rd residue aspartic acid to the 131st residue histidine or to the 130th residue cysteine in the amino acid sequence of SEQ ID NO:1.
